# EUROPEAN PATENT APPLICATION

(11) **EP 1 338 606 A1**
(43) Date of publication of application: **27.08.2003**
(21) Application number: 02003989.7
(22) Date of filing: 22.02.2002
(51) Int. Cl.: C07K 14/15, C12N 15/48, C12P 21/00, C12N 15/11, C07K 16/08, G01N 33/53, A61K 38/00

(54) **Nucleic Acid encoding an HERV-H polypeptide for diagnosing colorectal cancer**

(71) Applicant: Von Knebel Doeberitz, Magnus, 69120 Heidelberg (DE)
(72) Inventor: von Knebel Doeberitz, Magnus, 69118 Heidelberg (DE); Wentzensen, Nicolas, 69123 Heidelberg (DE); Gebert, Johannes, 69221 Dossenheim (DE); Linnebacher, Michael, 66578 Landsweiler-Reden (DE)
(74) Representative: Schüssler, Andrea, Dr.

(57) **Abstract**

HERV-H retroviral sequences that are preferentially expressed in human cancers, particularly in colorectal cancer, as well as polypeptides encoded by such nucleotide sequences and containing at least a portion of a tumor associated polypeptide. Vaccines and pharmaceutical compositions for prevention and treatment of cancer or precursors of cancer comprising such polypeptides, or nucleic acid molecules encoding such polypeptides, are also provided, together with DNA molecules for preparing the inventive polypeptides. Also provided herein are methods that can be used for the early detection, diagnosis, and monitoring of human cancers, particularly of colon cancers.

SEQUENCE LISTING

<110> Prof. Dr. v. Knebel-Doeberitz, Magnus

<120> Compounds for Diagnosis, Prevention and Therapy of Cancer

<130> K 3023EP

<140> EP 02 003 989.7
<141> 2002-02-22

<160> 27

<170> PatentIn version 3.1

<210> 1
<211> 822
<212> DNA
<213> Homo sapiens

<400> 1
atggacgcac atgaaatttg gtgccatgac tcggatcggg ggacctccct tgggagatca 60
atcctccgtc ctcctgctct ttgctccctg agaaagatcc acctacgacc tcaggtcctc 120
agaccgacga gcccaagaaa catctcacca atttcaaatc tggtaagcag cctcttttta 180
ctctcttctc caacttccct cactatccct caaccacttt ctcctttcaa tcttggcact 240
acacttcaat ctctcccttc tcttaatttc aattcctttc attttctggt agagacaaaa 300
gagacacgtt ttatccgtgg acccaaaact ccggcgctgg tcacagactg ggaaggcagc 360
cttcccttgg tgtttaatca ttgcagggat gcctctctga ttatacaccc acgtttcaag 420
ggtgtcagac cacgcaggga caactgcctt ggtccttcac ccttagcagc aagtcccgct 480
tttctgggga aggggcaagt accccaaccc cttctctccg tgtctctacc ccttctctgc 540
ttttccgggg acagggcaag taccccaacc ccttctctcc gtgtctctac tccttctctg 600
cttttctggg gacagggcaa gtaccccaac cccttctctc cttgtctcta ccccttctct 660
gcttttctgg ggcaggggca agtacccctc aaccccttct ccttcaccct tagcggtaag 720

tcccgctttt ctagggggca ggggcaagta ccccccaacc ccttctcctt cacccttagt    780

ggcaagtcct gctttcctgg ggcaggggca agtacccctt aa                        822

<210>  2

<211>  273

<212>  PRT

<213>  Homo sapiens

<400>  2

<210>  3

<211>  1650

<212>  DNA

<213>  Homo sapiens

<220>

<221>  CDS

<222>  (85)..(906)

<220>

<221>  primer_bind

<222>  (41)..(62)

<223>  (Binding site for 1st Primer)

<220>

<221>  primer_bind

<222>  (425)..(443)

<223>  (Binding site for 2nd Primer)

<220>

<221>  primer_bind

<222>  (952)..(971)

<223>  (Binding site for 3rd Primer)

<220>

<221>  primer_bind

<222>  (1599)..(1621)

<223>  (Binding site for 4th Primer)

<400>  3
ctcttttcgg actcagcccg cctgcaccca ggtgaaataa acagccatgt tgctcacaca      60
aagcctgttt ggtggtctct tcac atg gac gca cat gaa att tgg tgc cat      111
gac tcg gat cgg ggg acc tcc ctt ggg aga tca atc ctc cgt cct cct      159
gct ctt tgc tcc ctg aga aag atc cac cta cga cct cag gtc ctc aga      207
ccg acg agc cca aga aac atc tca cca att tca aat ctg gta agc agc      255
ctc ttt tta ctc tct tct cca act tcc ctc act atc cct caa cca ctt      303
tct cct ttc aat ctt ggc act aca ctt caa tct ctc cct tct ctt aat      351
ttc aat tcc ttt cat ttt ctg gta gag aca aaa gag aca cgt ttt atc      399
cgt gga ccc aaa act ccg gcg ctg gtc aca gac tgg gaa ggc agc ctt      447
ccc ttg gtg ttt aat cat tgc agg gat gcc tct ctg att ata cac cca      495
cgt ttc aag ggt gtc aga cca cgc agg gac aac tgc ctt ggt cct tca      543
ccc tta gca gca agt ccc gct ttt ctg ggg aag ggg caa gta ccc caa      591
ccc ctt ctc tcc gtg tct cta ccc ctt ctc tgc ttt tcc ggg gac agg      639
gca agt acc cca acc cct tct ctc cgt gtc tct act cct tct ctg ctt      687
ttc tgg gga cag ggc aag tac ccc aac ccc ttc tct cct tgt ctc tac 735
ccc ttc tct gct ttt ctg ggg cag ggg caa gta ccc ctc aac ccc ttc 783
tcc ttc acc ctt agc ggt aag tcc cgc ttt tct agg ggg cag ggg caa 831
gta ccc ccc aac ccc ttc tcc ttc acc ctt agt ggc aag tcc tgc ttt 879
cct ggg gca ggg gca agt acc cct taa ccccttctcc ttcaccctta 926
gcagcaagtc ccacttttct agggggcaag aaaccccaaa ccccttccct ccgtgtcttt 986
acgctctctt ttctctgggt ttgcttcctt cactatgggc aaccttccat cctccattcc 1046
tccttctccc ttagcctgtg tgctcaagaa cttaaaacct cttcaactca cacctgacct 1106
aaaacctaaa tgcctcattt tcttctgcaa caccgcttgg ccccaataca aacttgacaa 1166
tggctctaaa tggccagaaa atggcacttt cgatttctcc atcctacaag acctaaataa 1226
tttttgtcaa aaaatgggca aatggtctga ggtgcctgat gtccaggcat tcttttacac 1286
atccgtccct tcctagtctc tgtgtccagt gcactcgtcc caaatcttcc ttctttccct 1346
ccacctgtcc cctcagtccc aaccccaggc gttgctgagt gtgtctaatc ttccttttct 1406
acaaacccat ctgacctctc ccctcctctc cacgccaagc taggtcccaa ttcttcctca 1466
ggctccactc ctccaccctg taatcttttt atcacctccc ctcctcacac ctggtccggc 1526
ttacagtttc gttccgtgac tagccttccc ccacctgccc agcaatttac tcttaaaaag 1586
gtggctggag ctaaagacat agtcaaggtt aatgctcctt tttctttatc ccaaatcaga 1646
agcg 1650

<210> 4

<211> 273

<212> PRT

<213> Homo sapiens

<400> 4

<210>  5

<211>  21

<212>  DNA

<213>  Artificial Sequence

<220>

<223>  Description of the Artificial Sequence: Primer

<400>  5
cagccatgtt gctcacacaa a                                                21

<210>  6

<211>  21

<212>  DNA

<213>  Artificial Sequence

<220>

<223> Description of the Artificial Sequence: Primer

<400>  6
cagccctgtt gctcacacaa a                                                21

<210>  7

<211>  19

<212>  DNA

<213>  Artificial Sequence

<220>

<223>  Description of the Artificial Sequence: Primer

<400>  7
tcacagactg ggaaggcag                                                   19

<210>  8

<211>  19

<212>  DNA

<213>  Artificial Sequence

<220>

<223>  Description of the Artificial Sequence: Primer

<400>  8
aggggtttgg ggtttcttg                                                    19

<210>  9

<211>  22

<212>  DNA

<213>  Artificial Sequence

<220>

<223>  Description of the Artificial Sequence: Primer

<400>  9
gcattaacct tgactatgtc tt                                                22

<210>  10

<211>  991

<212>  DNA

<213>  Homo sapiens

<400>  10
taattttcca ttaccttccc aaatcctata aaacggcccc acccctatct ccctttgctg      60

actctctttt cggactcagc ccgcctgcac ccaggtgaaa taaacagcca tgttgctcac     120

acaaagcctg tttggtggtc tcttcacatg gacgcacatg aaatttggtg ccatgactcg     180

gatcggggga cctcccttgg gagatcaatc ctccgtcctc ctgctctttg ctccctgaga     240

aagatccacc tacgacctca ggtcctcaga ccgacgagcc caagaaacat ctcaccaatt     300

tcaaatctgg taagcagcct ctttttactc tcttctccaa cttccctcac tatccctcaa     360

ccactttctc ctttcaatct tggcactaca cttcaatctc tcccttctct taatttcaat     420

tcctttcatt ttctggtaga gacaaaagag acacgtttta tccgtggacc caaaactccg 480
gcgctggtca cagactggga aggcagcctt cccttggtgt ttaatcattg cagggatgcc 540
tctctgatta tacacccacg tttcaagggt gtcagaccac gcagggacaa ctgccttggt 600
ccttcaccct tagcagcaag tcccgctttt ctggggaagg ggcaagtacc ccaacccctt 660
ctctccgtgt ctctacccct tctctgcttt tccggggaca gggcaagtac cccaacccct 720
tctctccgtg tctctactcc ttctctgctt ttctggggac agggcaagta ccccaacccc 780
ttctctcctt gtctctaccc cttctctgct tttctggggc aggggcaagt acccctcaac 840
cccttctcct tcacccttag cggtaagtcc cgcttttcta gggggcaggg gcaagtaccc 900
cccaacccct tctccttcac ccttagtggc aagtcctgct ttcctggggc aggggcaagt 960
accccttaac cccttctcct tcacccttag c 991

<210> 11
<211> 976
<212> DNA
<213> Homo sapiens

<400> 11
taattttcct ttacctaccc aaatcctata aaatggcccc actcttatct cccttccctg 60
actctctttt cagactcagc ccgcctgcac ccaggtgaaa taaacagcca tgttgctcac 120
acaaagcctg tttggtggtc tcttcacacg gatgcacatg aaatttggtg ccgtgactcg 180
gatcggggga cctcccttgg gagatcaatc ccccgtcctc ctgctctttg ctccgtgaga 240
aagatccacc tacgacctca ggtcctcaga ccgaccagcc caatgaacat ctcaccaatt 300
ttaaatcagg taagcagcct cttcttactc tcttctccaa cctctcactg tccctcaacc 360
actttctcct ttccactctt caatctctcc cttctcttaa tttcaattcc tttcattttc 420
tgggagagac aaaggagaca cattttatcc gtggacccaa aactccggcg ctggtcacag 480
actgggaagg cagccttccc ttggtgttta atcattgcag ggacacctct ctgattattc 540
acccatgttt caaaggtgtc agaccatgca gtgatgccta ccttggtcct tcaccctcag 600
cagcaagtcc cgcttttctg gggaaggggc aagtacccca acctcttctc tccttttctc 660
taccccttct ctgctttcct ggggcagggg caagtacccc ccaacccttt tctccttcac 720
ccttagcggc aagtcctgct ttcctggggc aggggcaagt acccctcaac cccttctctt 780
tcacccttag tggcaagtcc cgcttttcta gggggaaaga acccccagtc ccttatttct 840
gcaccccaac ctcgtatctc tgtgccccaa tcccttattt ccatgccccg accccttatt 900
tccgtgcccc aaccccttat ttctgtgccc tatcccttat ttccatgccc caacctctta 960
tctctgcacc ccaacc 976

<210>  12

<211>  10

<212>  PRT

<213>  Artificial Sequence

<220>

<223>  Description of the Artificial sequence: HLA-A0201 restricted Pept
       ide

<400>  12

Ile Leu Arg Pro Pro Ala Leu Cys Ser Leu
1               5                   10

<210>  13

<211>  10

<212>  PRT

<213>  Artificial Sequence

<220>

<223>  Description of the Artificial sequence: HLA-A0201 restricted Pept
       ide

<400>  13

Ala Leu Val Thr Asp Trp Glu Gly Ser Leu
1               5                   10

<210>  14

<211>  10

<212>  PRT

<213>  Artificial Sequence

<220>

<223>  Description of the Artificial sequence: HLA-A0201 restricted Pept
       ide

<400>  14

Pro Leu Leu Ser Val Ser Leu Pro Leu Leu
1               5                   10

<210>  15

<211>  10

<212>  PRT

<213>  Artificial Sequence

<220>

<223>  Description of the Artificial sequence: HLA-A0201 restricted Pept
       ide

<400>  15

Ser Leu Asn Phe Asn Ser Phe His Phe Leu
1               5                   10

<210>  16

<211>  10

<212>  PRT

<213>  Artificial Sequence

<220>

<223>  Description of the Artificial sequence: HLA-A0201 restricted Pept
       ide

<400>  16

Phe Leu Leu Ser Ser Pro Thr Ser Leu Thr
1               5                   10

<210>  17

<211>  9

<212>  PRT

<213>  Artificial Sequence

<220>

<223>  Description of the Artificial sequence: HLA-A0201 restricted Pept
       ide

<400>  17

Leu Leu Ser Val Ser Leu Pro Leu Leu
1               5

<210>  18

<211>  9

<212>  PRT

<213>  Artificial Sequence

<220>

<223>  Description of the Artificial sequence: HLA-A0201 restricted Pept
       ide

<400>  18

Ser Leu Arg Val Ser Thr Pro Ser Leu
1               5

<210>  19

<211>  9

<212>  PRT

<213>  Artificial Sequence

<220>

<223>  Description of the Artificial sequence: HLA-A0201 restricted Pept
       ide

<400>  19

Cys Leu Tyr Pro Phe Ser Ala Phe Leu
1               5

<210>  20

<211>  9

<212>  PRT

<213>  Artificial Sequence

<220>

<223>  Description of the Artificial sequence: HLA-A0201 restricted Pept
       ide

<400>  20

Phe Leu Leu Ser Ser Pro Thr Ser Leu
1               5

<210>  21

<211>  9

<212>  PRT

<213>  Artificial Sequence

<220>

<223>  Description of the Artificial sequence: HLA-A0201 restricted Pept
       ide

<400>  21

Asn Leu Val Ser Ser Leu Phe Leu Leu
1               5

<210>  22

<211>  19

<212>  DNA

<213>  Artificial Sequence

<220>

<223>  Description of the Artificial Sequence: Primer

<400>  22
tcacagactg ggaaggcag                                                    19

<210>  23

<211>  19

<212>  DNA

<213>  Artificial Sequence

<220>

<223>  Description of the Artificial Sequence: Primer

<400>  23
aggggtttgg ggtttcttg                                                    19

<210>  24

<211>  20

<212>  DNA

<213>  Artificial Sequence

<220>

<223>  Description of the Artificial Sequence: Primer

<400>  24
cacgttttat ccgtggaccc                                                   20

<210>  25

<211>  21

<212>  DNA

<213>  Artificial Sequence

<220>

<223>  Description of the Artificial Sequence: Primer

<400>  25
aggcatccct gcaatgatta a                                                 21

<210>  26

<211>  16

<212>  DNA

<213>  Artificial Sequence

<220>

<223>  Description of the Artificial Sequence: Primer

<400>  26
ccggcgctgg tcacag                                                       16

<210>  27

<211>  25

<212>  DNA

<213>  Artificial Sequence

<220>

<223>  Description of the Artificial Sequence: Primer

<400>  27
tgaaacgtgg gtgtataatc agaga                                          25

## Description

The present invention relates to a nucleotide sequence that is preferentially expressed in human cancer tissues and a polypeptide encoded by such nucleotide sequence. The inventive nucleotide sequence and polypeptide may be used as target molecules for cancer early detection, staging and diagnosis as well as in vaccines and pharmaceutical compositions for the prevention and treatment of cancer, particularly of colorectal cancer.

Despite significant scientific and medical research efforts, neoplastic diseases still remain a major cause of human mortality. One major reason for this unsatisfying situtation is, that most neoplastic diseases are diagnosed at relatively late stages, when isolated tumor cells or small tumor cell aggregates were already released from the primary tumor and distributed in the whole organism of the host and might have eventually already caused occult or frank metastatic disease. Early cancers and in particular precancers usually do not cause any symptoms and are not realized by the respective patients. To overcome this, more research efforts and clinical programs are required to improve cancer early detection technologies, as well as to develop true preventive or therapeutic vaccination strategies to immunize patients either before a defined cancer emerged or after resection of a cancer or its precursors to prevent survival of disseminated isolated cancer cells (DTCs) which might have been released from the neoplasm either before or during primary surgical intervention. For few cancers, in particular cancer of the uterine cervix efficient cancer early detection programs could be established. The subsequent reduction of mortality rates associated with these specific neoplasms convincingly demonstrated the high effectiveness of the early detection programs. Preventive and therapeutic vaccination programs proved to be effective particularly for those cancers, in which a causative infectious agent could be identified and for which the infection could be prevented by a vaccine. In other clinical settings, adoptive transfer of cytotoxic T-cells directed against defined viral or cancer associated antigens showed high therapeutic effectiveness. This suggests that at least in some cancers tumor specific antigens might stimulate specific immune responses, which might prove helpful in the prevention and/or treatment of neoplastic diseases.

Colorectal cancer is the second most frequently diagnosed malignancy in the Western countries and the second most common cause of cancer related deaths. The five-year survival rate for patients with colorectal cancer detected in an early stages is relatively good whereas it is very poor in advanced stages, particularly, when distant metastasis already emerged. Currently, early detection of colorectal cancers relies on fairly unspecific tests as for example the detection of occult blood in fecal specimens. This test is hampered by many false positive and false negative test results and has therefore only limited specificity and sensitivity. Accordingly, there remains a clear need in the art for early detection and diagnostic techniques as well as treatment methods and improved vaccines for colon cancer.

Human endogenous retroviral elements (HERV) account for approximately 8,3% of the human genome. They have developed in the course of evolution by integration of exogenous retroviruses into the germ line. The major part of integrations happened about 30-45 million years ago. HERVs are subdivided into three classes, based on their respective exogenous retrovirus ancestors. Class I HERVs belong to the gamma retroviruses, class II to the beta retroviruses and class III to the Spumaviruses (Pringle 1999). HERVs are further subclassified according to their distinct primer binding site (PBS) that can bind tRNAs carrying specific amino acids.

These tRNAs serve as a primer for reverse transcription in the replication of exogenous retroviruses. Like their protoviruses, the endogenous retroviruses usually consist of three genes, which are expressed under control of the long terminal repeat (LTR).: gag, pol and env. Gag is coding for matrix and capsid proteins, pol is coding for reverse transcriptase and integrase proteins, necessary for integration and replication. The env gene products build the viral envelope and transmembrane structures. Usually, because of their long persistence in the host genome without selective pressure, these genes are mutated in endogenous retroviruses and have no coding potential. There are some examples where open reading frames (ORF) with a coding potential were found in HERVs. Copies of HERV-H and HERV-W were shown to contain open env reading frames (Lindeskog 1999, Blond 1999) and Mayer et al. described a member of the HERV-K family with open env and gag reading frames and only one mutation in pol (Mayer 1999). Berkhout et al. found an active RT enzyme, which is coded by the pol gene, deriving from a copy of HERV-K (Berkhout 1999). Class II HERVs may harbour supplementary genes: A dUTPase gene between the gag and pol genes was described for HERV-HML5 (Tristem 2000). In some of the HERV-K copies, an additional ORF was found in the area of the env gene. This ORF was called c-orf and was shown to be a functional homologue without any sequence similarity to the HIV rev gene (Yang 1999).

The HERV LTRs are strong promoters which are capable of disturbing the regulation of host gene expression. Some LTRs have a cell type specific activity (Schön 2001). LTRs were also shown to have binding sites for human gene regulatory factors, such as myb (de Parsefal 1999) and sp1 (Sjottem 1996). LTR activity can be altered by the host genome through methylation, a cellular defense mechanism against foreign DNA integration (Abrink 1998). HERV sequences might also affect cellular splicing mechanisms, as it was described in teratocarcinoma cell lines (Kowalski 1999).

A lot of studies have been performed on the relation between HERVs and malignant diseases. Activation of HERVs in tumors was described for human HERV-K, HERV-H, MMTV, HERV-E, HERV-A and for ERVs in mouse and rat (Yang 2000, Guillem 1988). HERV-K genes were found expressed mainly in teratocarcinoma (Lower 1993, Bieda 2001), but also in other cell lines (Kim 2001) and in germ cell tumors (Herbst 1999) by several groups. HERV-K /HTDV were described to be higher expressed in testicular tumors as compared to normal testis, and an immune reaction of patients against HERV gag and env proteins was observed, that resolved after tumor resection (Boller 1997). HERV-K env was also found expressed in breast cancer cell lines and in primary tumor samples from breast cancer (Wang-Johanning 2001). Willer et al (Willer 1997) did not find characteristic transcription patterns of HERV-K in hematologic and solid tumors compared to normal tissue.

It was shown that the env gene of HERV-H has immunosupressive effects in mice: Tumor cells that were normally rejected after injection grew out to tumors if HERV env was expressed in these animals. The same effect was observed using env proteins of the exogenous Moloney murine leukemia virus (Mangeney 1998, Mangeney 2001). Lindeskog et al (Lindeskog 1997) showed different HERV-H splice variants in T-cell leukemia versus normal lymphocytes.

Melana et al. (Melana 2001) showed expression of MMTV env genes in 32/106 breast cancer samples compared to 1/106 matched normal samples. Liu et al (Liu 2001) identified proviral MMTV structures in samples from two independent breast cancer cases.

The activiation of a HERV-A LTR in a bronchial carcinoma cell line was found by Tomita et al. (Tomita 1990), the authors could not amplify this transcript in control tissue. Turbeville et al (Turbeville 1997) generated a polyclonal antibody against the transmembrane protein (part of env) of HERV-E. This antibody reacted against a 58 kDA Protein found in colon cancer, prostate cancer, endothelial and seminomal cell lines and peripheral blood mononuclear cells (PBMCs).

Thus, the technical problem underlying the present invention is to provide a meaningful specific marker which correlates with cancer and its precursors, preferably colorectal cancer, and which can be used for cancer early detection, diagnosis, prevention and treatment.

The solution to said technical problem is achieved by providing the embodiments characterised in the claims. The present invention provides such a marker, i.e. a particular nucleic acid molecule and a polypeptide encoded thereof, which is expressed in colon cancers. In the study leading to the present invention a member of the HERV (human endogenous retrovirus)-H family located on Chr. Xp was found. The member of the HERV-H family found during the experiments resulting in the present invention contains 5' and 3' LTRs, a gag region, a pol region and a small part of env. A major part of env is deleted as compared to published full length HERV-H sequences. The viral gag, pol and env genes were defective in the isolated HERV-H sequence but, in contrast to the other sequences, it contained a new, up to now not described open reading frame (ORF) in the 5' region. This 5' ORF starts directly after the LTR and uses the second ATG after the transcription initiation site. It could be shown that the specific sequence from this HERV-H is transcribed and encodes a novel protein.

Expression analysis was performed using standard RT-PCR protocols and real-time-PCR. In all cases, matched pairs of tumor and corresponding normal tissue was analyzed. In total, 5/14 colon cancers analyzed by standard RT-PCR showed a differential activation of the HERV-H ORF sequence and 3/5 colon cancer analyzed by real-time-PCR showed activation of the sequence in the tumor.

### Brief description of the drawings

Figure 1: Nucleotide sequence of the isolated HERV-H open reading frame located on chromosome Xp from the start ATG to the stop TAA (822 nucleotides)
Figure 2: Amino acid sequence of the predicted protein derived from the HERV-H open reading frame on chromosome Xp Translation was performed using the "translate" program included in the Heidelberg Unix Sequence Analysis Resource (HUSAR) on the basis of the standard mammalian genetic code.

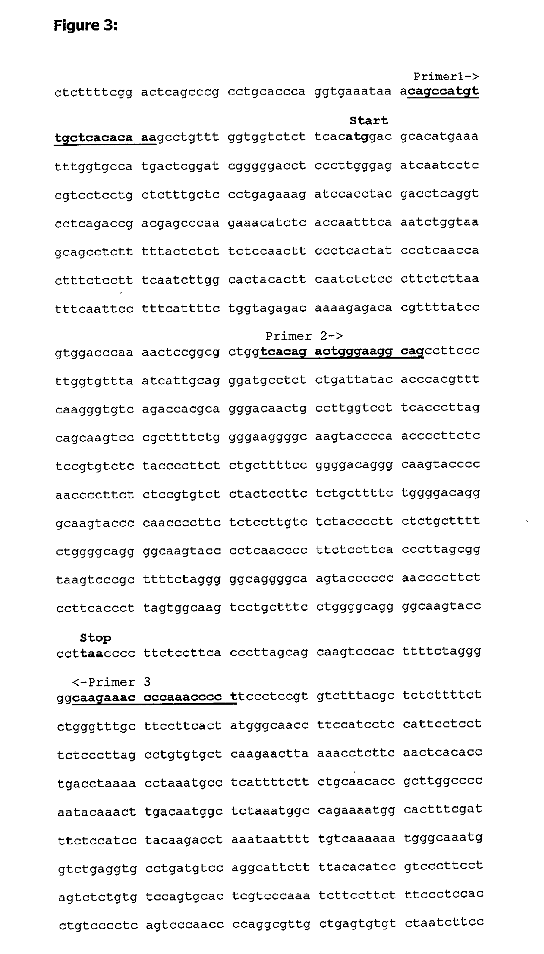

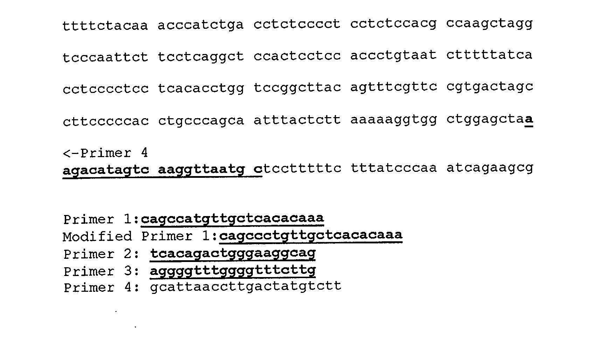

Figure 3: Genomic sequence containing the HERV-H ORF disclosed herein. The primer binding sites for the primers used in the RT-PCR experiments performed in the studies leading to the present invention are indicated within the figure. Modified Primer 1 and Primer 3 were used to construct the nucleic acid construct deposited at the DSMZ under DSM 14799. Primer 4 was used for RT; Primer 2 and 3 were used for PCR. All primers are listed from 5'->3' sequence. Target DNA only listed as sense strand.

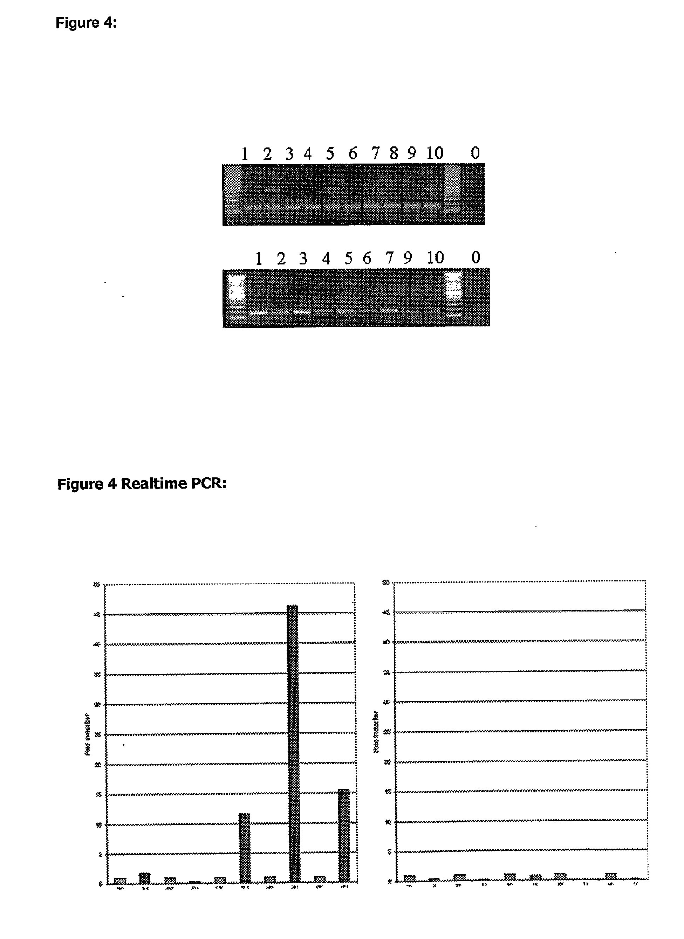

Figure 4: Results of analytical agarose gel electrophoresis of the products of RT-PCR experiments
   The experiments were performed using 10 representative tumor samples. The upper gel shows the 10 tumor samples; the lower gel shows 9 corresponding normal tissue samples as control samples. There was no appropriate normal tissue available from sample 8. The negative control is indicated by 0. There is a strong signal at 550 bp in the case of the tumor samples 2, 5 and 10 and a weaker signal can be observed in sample 1. No signals can be detected in the normal tissue samples.

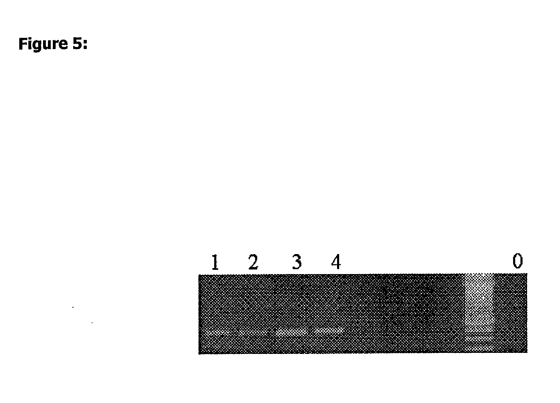

Figure 5: Qualitative agarose gel electrophoresis of four HERV-H ORF positive cell lines. The expression of the HERV-H ORF was determined using RT-PCR. The results were analysed by agarose gel electrophoresis. Lane 1: SiHa (Cervical Cancer) Lane 2: KM12 (Colon Cancer) Lane 3: Colo60H (Colon Cancer) Lane 4: LS174T (Colon Cancer) Lane 0: negative control.

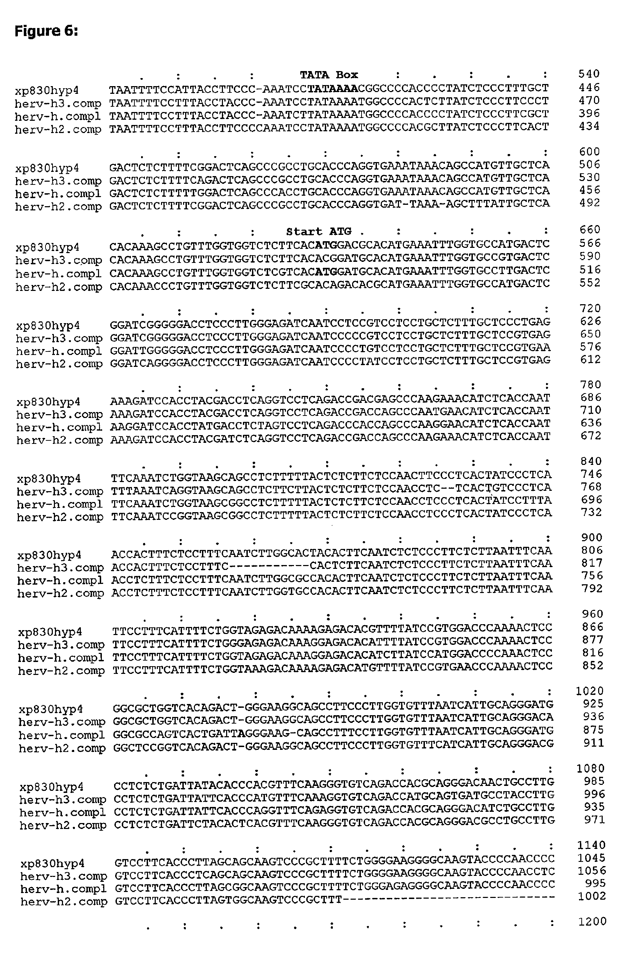

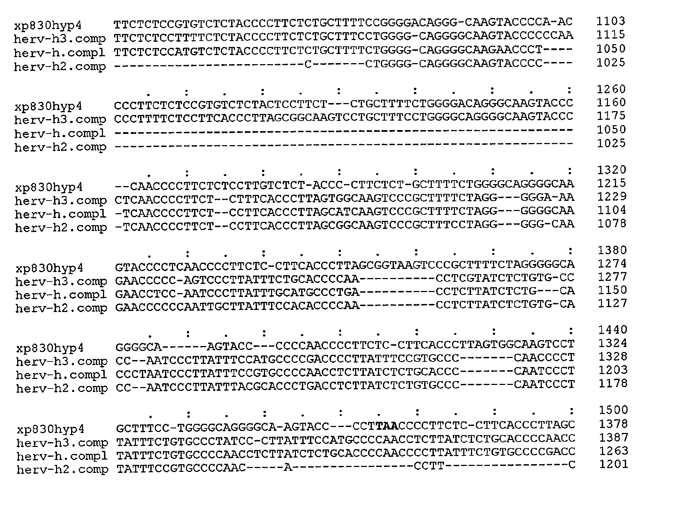

Figure 6: Multiple alignment of the open reading frame region of some HERV-H. xp830hyp4 is the sequence of the isolated HERV-H sequence from Xp. herv-h3.comp is a HERV-H sequence from PAC 271G9 on chromosome 6. herv-h.compl is a different HERV-H sequence, accession number D11078. herv-h2.comp is another HERV-H sequence, accession number GI:8439395. TATA-Box, Start and Stop codon of the open reading frame are indicated. Although there is a high similarity between the HERV-H sequences in that region, only xp830hyp4 shows an open reading frame.

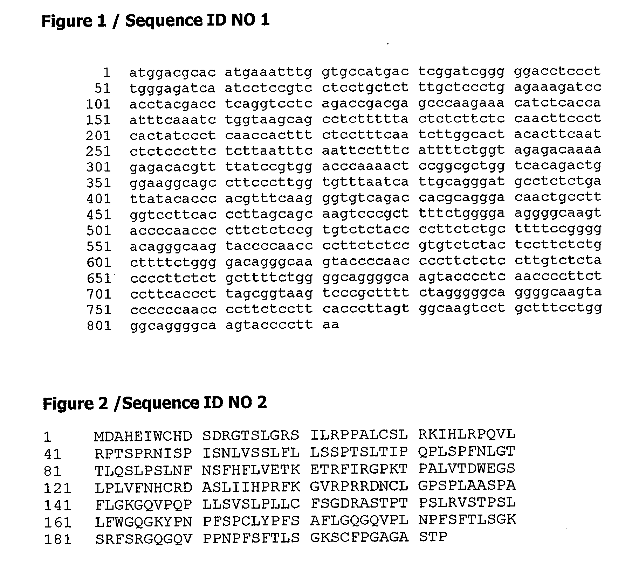

The present invention provides a nucleic acid molecule encoding a cancer-associated HERV-H polypeptide or a polypeptide exhibiting a biological property of a cancer-associated HERV-H polypeptide and being selected from the group consisting of
(a) a nucleic acid molecule encoding a polypeptide that comprises the amino acid sequence as depicted in Figure 2;
(b) a nucleic acid molecule comprising the coding region of the nucleotide sequence as depicted in Figure 1;
(c) a nucleic acid molecule included in DSMZ Deposit No. DSM 14799;
(d) a nucleic acid molecule the nucleic acid sequence of which deviates from the nucleic acid sequences specified in (b) to (c)due to the degeneration of the genetic code; and
(e) a nucleic acid molecule, which encodes a fragment or variant of a polypeptide that comprises the amino acid sequence depicted in Figure 2.

As used herein, a polypeptide exhibiting a biological property of a cancer-associated HERV-H polypeptide is understood to be a polypeptide exhibiting a leucine zipper domain and thus potentially showing DNA binding properties, wherein the activities of the polypeptide comprise e.g. the activity to modulate gene expression, to stimulate the proliferation of cells, to act in inhibition of apoptosis, to induce dedifferentiation of cells and/or to induce a tumor in an animal etc.

In a first embodiment, the invention provides an isolated nucleic acid molecule encoding a cancer-associated HERV-H polypeptide that comprises the amino acid sequence as depicted in Figure 2.

The present invention also provides a nucleic acid molecule encoding a cancer-associated HERV-H polypeptide comprising the nucleotide sequence as depicted in Figure 1.

The present invention provides not only the generated nucleotide sequence identified in Figure 1 and the predicted translated amino acid sequence identified in Figure 2 but also plasmid DNA containing a HERV-H cDNA deposited with the DSMZ, under DSM 14799. The nucleotide sequence of the deposited HERV-H ORF clone can readily be determined by sequencing the deposited clone in accordance with known methods. The predicted amino acid sequence can then be verified from such deposit. Moreover, the amino acid sequence of the polypeptide encoded by the deposited clone can also be directly determined by peptide sequencing or by expressing the protein in a suitable host cell containing the deposited HERV-H ORF encoding DNA, collecting the protein, and determining its sequence.

The nucleic acid molecules of the invention can be both DNA and RNA molecules. Suitable DNA molecules are, for example, genomic or cDNA molecules. It is understood that all nucleic acid molecules encoding all or a portion of a cancer-associated HERV-H polypeptide are also included, as long as they encode a polypeptide with one or more biological activities of said HERV-H polypeptide. The nucleic acid molecules of the invention can be isolated from natural sources or can be synthesized according to known methods.

The nucleic acid molecules of the present invention also include molecules which differ from the nucleic acid molecules with sequences shown in Figure 1 due to the degeneracy of the genetic code.

In a further embodiment, the present invention provides nucleic acid molecules which encode a fragment or variant of a polypeptide of the invention. The term "variant" as used herein relates to a cancer-associated HERV-H polypeptide according to the present invention showing at least one of its biological activities but differing in amino acid sequence compared to the amino acid sequence shown in Figure 2 due to deletion, substitution and/or insertions of amino acid residue(s). The amino acid sequence of said variant shows an identity of at least 70%, in particular an identity of at least 90%, preferably of at least 95% and, particularly preferred, of at least 98% to the amino acid sequence of the polypeptide of Figure 2. In order to identify and isolate nucleic acid molecules encoding such variants the molecules of the invention or parts of these molecules or the reverse complements of these molecules can be used, for example by means of hybridization. As a hybridization probe nucleic acid molecules can be used, for example, that have exactly or basically the nucleotide sequence as depicted in Figure1 or parts of these sequences.

The term "fragment" as used herein relates to a fragment of a cancer-associated HERV-H polypeptide according to the present invention. The fragment is encoded by a nucleic acid molecule which hybridizes specificially to nucleic acid molecules of the present invention but not to transcripts of ohter HERV sequences.

These nucleic acid molecules can be used, for example, as probes or primers in the diagnostic assay and/or kit described below and, preferably, are oligonucleotides having a length of at least 10, in particular of at least 15 and particularly preferred of at least 50 nucleotides. The nucleic acid molecules and oligonucleotides of the invention can also be used, for example, as primers for nucleic acid based amplification reactions such as for example polymerase chain reaction.

These fragments used can be synthetic fragments that were produced by means of conventional synthetic methods and the sequence of which basically corresponds to the sequence of a nucleic acid molecule of the invention.

As used herein, the term "hybridization" has the meaning of hybridization under conventional hybridization conditions, preferably under stringent conditions as described, for example, in Sambrook et al., Molecular Cloning, A Laboratory Manual 2^{nd} edition (1989) Cold Spring Harbor Laboratory Press, Cold Spring Harbor, NY.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The polypeptids encoded by the various variants of the nucleic acid molecules of the invention show certain common characteristics, such as at least one biological activity, molecular weight, immunological reactivity or conformation or physical properties like the electrophoretical mobility, chromatographic behavior, sedimentation coefficients, solubility, spectroscopic properties, stability, pH optimum, temperature optimum.

The invention furthermore relates to vectors containing the nucleic acid molecules of the invention. Preferably, they are plasmids, cosmids, viruses, bacteriophages and other vectors usually used in the field of genetic engineering. Vectors suitable for use in the present invention include, but are not limited to the T7-based expression vector for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an mRNA in prokaryotic and/or eukaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the nucleic acid molecules or vectors of the invention. A host cell is understood to be an organism that is capable to take up in *vitro* recombinant DNA and, if the case may be, to synthesize the polypeptides encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

A further embodiment of the invention relates to a polypeptide exhibiting a biological property of a cancer-associated HERV-H polypeptide and being encoded by the nucleic acid molecules of the invention, as well as to methods for their production, whereby, e.g., a host cell of the invention is cultivated under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations using, e.g., a specific antibody, or, e.g., can be substantially purified by the one-step method described in Smith and Johnson, Gene 67; 31-40 (1988). These polypeptides, however, not only comprise recombinantly produced polypeptides but include isolated naturally occurring polypeptides, synthetically produced polypeptides, or polypeptides produced by a combination of these methods. Means for preparing such polypeptides or related polypeptides are well understood in the art. These polypeptides are preferably in a substantially purified form.

It can be expected that, e.g., the inhibition of expression of the polypeptide of the present invention is useful for therapy. Accordingly, in a further preferred embodiment, the present invention relates to an antisense RNA sequence characterized in that it is reverse-complementary to an mRNA transcribed from the nucleic acid molecule of the present invention or a part thereof and (a) can selectively bind to said mRNA but (b) does not hybridize to transcripts of other HERV sequences, said antisense RNA being capable of inhibiting the synthesis of the polypeptide encoded by said nucleic acid molecules, and a ribozyme characterized in that (a) it is complementary to an mRNA transcribed from the nucleic acid molecule of the present invention or a part thereof but (b) does not hybridize to transcripts of other HERV sequences and can selectively bind to and cleave said mRNA, thus inhibiting the synthesis of the polypeptide encoded by said nucleic acid molecules. Ribozymes which are composed of a single RNA chain are RNA enzymes, i.e. catalytic RNAs, which can intermolecularly cleave a target RNA, for example the mRNA transcribed from the gene shown in Figure 1. It is now possible to construct ribozymes which are able to cleave the target RNA at a specific site by following the strategies described in the literature. (see, e.g., Tanner et al., in: Antisense Research and Applications, CRC Press Inc. (1993), 415-426). The two main requirements for such ribozymes are the catalytic domain and regions which are complementary to the target RNA and which allow them to bind to its substrate, which is a prerequisite for cleavage. Said complementary sequences, i.e., the antisense RNA or ribozyme, are useful for repression of expression, e.g. in the case of the treatment of a cell proliferative disorder associated with tumors, particularly colon cancer. Preferably, the antisense RNA and ribozyme of the invention are complementary to the coding region of the mRNA transcribed from the nucleic acid sequence of Figure 1, e.g. to the 5' part of the coding region. The person skilled in the art provided with the sequences of the nucleic acid molecules of the present invention will be in a position to produce and utilize the above described antisense RNAs or ribozymes. The region of the antisense RNA and ribozyme, respectively, which shows complementarity to the mRNA transcribed from the nucleic acid molecules of the present invention preferably has a length of at least 10, in particular of at least 15 and particularly preferred of at least 50 nucleotides.

The present invention also relates to binding agents that specifically bind to a cancer-associated HERV-H polypeptide or a related polypeptide as defined above. The term binding agent comprises a variety of substances such as oligopeptides, antibodies, peptidomimetic molecules comprising antigen binding oligopeptides, nucleic acids, carbohydrates, organic compounds, etc.. Antibody, preferably, relates to antibodies which consist essentially of pooled monoclonal antibodies with different epitopic specificities, as well as distinct monoclonal antibody preparations. Monoclonal antibodies are made from antigen containing fragments of the polypeptides of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies of the present invention include chimerical, single chain, and humanized antibodies.

Binding agents according to the present invention may for example be employed for the inhibition of the activity of the HERV-H polypeptides disclosed herein. Binding agents which are useful for this inhibtion may comprise nucleic acids (DNA, RNA, PNA etc.), polypeptides (antibodies, receptors, antigen-binding fragments, oligopeptides), carbohydrates, lipids, organic or inorganic compounds (metal-ions, sulfur compounds, boranes, silicates, reducing agents, oxidizing agents). The binding agents may preferably interact with the polypeptide by binding to epitopes, that are essential for the biological activity. The interaction may be reversible or irreversibly. The binding may be noncovalent or even covalent binding to the polypeptide. Furthermore the binding agents may introduce alterations to the polypeptide, that alter or diminish the biological activity of the inventive polypeptide.

For certain purposes, e.g. diagnostic methods, the binding agents of the present invention, e.g. the antibodies can be detectably labeled, for example, with a radioisotope, a bioluminescent compound, a chemiluminescent compound, a fluorescent compound, a metal chelate, or an enzyme. Furthermore any method suitable for the detection of the intermolecular interaction may be employed.

In a further aspect, the present invention, relates to a method for identifying a binding partner to a cancer-associated HERV-H polypeptide of the invention (or related polypeptide) of the invention comprising:
(a) contacting a cancer-associated HERV-H polypeptide of the invention with a compound to be screened; and
(b) determining whether the compound effects an activity of the polypeptide.

A cancer-associated HERV-H polypeptide of the invention may be used to screen for proteins or other compounds that bind to a cancer-associated HERV-H polypeptide of the invention or for proteins or other compounds to which said polypeptide binds. The binding of the cancer-associated HERV-H polypeptide of the invention and the molecule may activate (agonist), increase, inhibit (antagonist), or decrease activity of the cancer-associated HERV-H polypeptide of the invention or the molecule bound. Examples of such molecules include antibodies, oligonucleotides, proteins (e.g., receptors), or small molecules.

Preferably, the molecule is closely related to a natural ligand of a cancer-associated HERV-H polypeptide of the invention, e.g., a fragment of the ligand, or a natural substrate, a ligand, a structural or functional mimetic; see, e.g., Coligan, Current Protocols in Immunology 1(2) (1991); Chapter 5. Similarly, the molecule can be closely related to the natural receptor to which a cancer-associated HERV-H polypeptide of the invention might bind, or at least, a fragment of the receptor capable of being bound by a cancer-associated HERV-H polypeptide of the invention (e.g., active site). In either case, the molecule can be rationally designed using known techniques.

Preferably, the screening for these molecules involves producing appropriate cells which express a cancer-associated HERV-H polypeptide of the invention, either as a secreted protein or on the cell membrane. Preferred cells include cells from mammals, yeast, Drosophila, or E. coli. Cells expressing said polypeptide (or cell membrane containing the expressed polypeptide) are then preferably contacted with a test compound potentially containing the molecule to observe binding, stimulation, or inhibition of activity of a cancer-associated HERV-H polypeptide of the invention.

The assay may simply test binding of a candidate compound to a cancer-associated HERV-H polypeptide of the invention, wherein binding is detected by a label, or in an assay involving competition with a labeled competitor. Further, the assay may test whether the candidate compound results in a signal generated by binding to a cancer-associated HERV-H polypeptide of the invention.

Alternatively, the assay can be carried out using cell-free preparations, polypeptide/molecule affixed to a solid support, chemical libraries, or natural product mixtures. The assay may also simply comprise the steps of mixing a candidate compound with a solution containing a cancer-associated HERV-H polypeptide of the invention, measuring HERV-H polypeptide/molecule activity or binding, and comparing the HER-V polypeptide/molecule activity or binding to a standard.

Preferably, an ELISA assay can measure the level of a cancer-associated HERV-H polypeptide of the invention or activity in a sample (e.g., biological sample) using a monoclonal or polyclonal antibody. The antibody can measure HERV-H polypeptide level or activity by either binding, directly or indirectly, to said polypeptide or by competing with said polypeptide for a substrate. All of these above assays can be used as diagnostic or prognostic markers. The molecules discovered using these assays can be used to treat disease or to bring about a particular result in a patient (e.g., elimination of a tumor or stop of progression of tumor growth) by activating or inhibiting the HERV-H molecule. Moreover, the assays can discover agents which may inhibit or enhance the production of the cancer-associated HERV-H polypeptide of the invention from suitably manipulated cells or tissues.

Therefore, the invention includes a method of identifying compounds which bind to a cancer-associated HERV-H polypeptide of the invention comprising the steps of: (a) incubating a candidate binding compound with a polypeptide of the invention; and (b) determining if binding has occurred.

Moreover, the invention includes a method of identifying activators/agonists or inhibitors/antagonists of a cancer-associated HERV-H polypeptide of the invention comprising the steps of: (a) incubating a candidate compound with a polypeptide of the invention; b) assaying a biological activity, and (c) determining if a biological activity of the polypeptide of the invention has been altered.

In a further embodiment, the present invention relates to a method of identifying and obtaining a drug candidate for therapy of a cancer, preferably colon cancer, comprising the steps of:
(a) contacting a polypeptide of the present invention or a cell expressing said polypeptide in the presence of components capable of providing a detectable signal in response to interaction of said polypeptide with said drug candidate to be screened, and
(b) detecting presence or absence of a signal or increase of the signal generated, wherein the presence or increase of the signal is indicative for a putative drug.

For example, the determination of HERV-H polypeptide - DNA interaction, the determination of transcriptional activation of certain genes (e.g. by differential analysis of mRNA expression), the detection of changes in cell proliferation or cell differentiation properties can be used to measure a biological activity of a cancer-associated HERV-H polypeptide of the invention.

The drug candidate may be a single compound or a plurality of compounds. The term "plurality of compounds" in a method of the invention is to be understood as a plurality of substances which may or may not be identical.

Said compound or plurality of compounds may be chemically synthesized or microbiologically produced and/or comprised in, for example, samples, e.g., cell extracts from, e.g., plants, animals or microorganisms. Furthermore, said compound(s) may be known in the art but hitherto not known to be capable of suppressing or activating cancer-associated HERV-H polypeptides of the invention. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994). The plurality of compounds may be, e.g., added to the reaction mixture, culture medium, injected into a cell or otherwise applied to a transgenic animal. The cell or tissue that may be employed in the method of the invention preferably is a host cell or mammalian cell of the invention described in the embodiments hereinbefore.

If a sample containing a compound or a plurality of compounds is identified in the method of the invention, then it is either possible to isolate the compound from the original sample identified as containing the compound capable of suppressing or activating a cancer-associated HERV-H polypeptide of the invention, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical.

Several methods are known to the person skilled in the art for producing and screening large libraries to identify compounds having specific affinity for a target. These methods include the phage-display method in which randomized peptides are displayed from phage and screened by affinity chromatography to an immobilized receptor; see, e.g., WO 91/17271, WO 92/01047, US-A-5,223,409. In another approach, combinatorial libraries of polymers immobilized on a chip are synthesized using photolithography; see, e.g., US-A-5,143,854, WO 90/15070 and WO 92/10092. The immobilized polymers are contacted with a labeled receptor and scanned for label to identify polymers binding to the receptor. The synthesis and screening of peptide libraries on continuous cellulose membrane supports that can be used for identifying binding ligands of the polypeptide of the invention and thus possible inhibitors and activators is described, for example, in Kramer, Methods Mol. Biol. 87 (1998), 25-39. This method can also be used, for example, for determining the binding sites and the recognition motifs in the polypeptide of the invention. In like manner, the substrate specificity of the DnaK chaperon was determined and the contact sites between human interleukin-6 and its receptor; see Rudiger, EMBO J. 16 (1997), 1501-1507 and Weiergraber, FEBS Lett. 379 (1996), 122-126, respectively. Furthermore, the above-mentioned methods can be used for the construction of binding supertopes derived from the polypeptide of the invention. A similar approach was successfully described for peptide antigens of the anti-p24 (HIV-1) monoclonal antibody; see Kramer, Cell 91 (1997), 799-809. A general route to fingerprint analyses of peptide-antibody interactions using the clustered amino acid peptide library was described in Kramer, Mol. Immunol. 32 (1995), 459-465. In addition, antagonists of the polypeptide of the invention can be derived and identified from monoclonal antibodies that specifically react with the polypeptide of the invention in accordance with the methods as described in Doring, Mol. Immunol. 31 (1994), 1059-1067.

More recently, WO 98/25146 described further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with the polypeptides according to the invention or nucleic acid molecules encoding such molecules are, for example, the in vitro screening with the phage display system as well as filter binding assays or "real time" measuring of interaction using, for example, the BIAcore apparatus (Pharmacia).

All these methods can be used in accordance with the present invention to identify activators/agonists and inhibitors/antagonists of the cancer-associated HERV-H polypeptide of the invention or related polypeptide.

Various sources for the basic structure of such an activator or inhibitor can be employed and comprise, for example, mimetic analogs of the polypeptide of the invention. Mimetic analogs of the polypeptide of the invention or biologically active fragments thereof can be generated by, for example, substituting the amino acids that are expected to be essential for the biological activity with, e.g., stereoisomers, i.e. D-amino acids; see e.g., Tsukida, J. Med. Chem. 40 (1997), 3534-3541. Furthermore, in case fragments are used for the design of biologically active analogs pro-mimetic components can be incorporated into a peptide to reestablish at least some of the conformational properties that may have been lost upon removal of part of the original polypeptide; see, e.g., Nachman, Regul. Pept. 57 (1995), 359-370. Furthermore, the polypeptide of the invention can be used to identify synthetic chemical peptide mimetics that bind to or can function as a ligand, substrate, binding partner or the receptor of the polypeptide of the invention as effectively as does the natural polypeptide; see, e.g., Engleman, J. Clin. Invest. 99 (1997), 2284-2292. For example, folding simulations and computer redesign of structural motifs of the polypeptide of the invention can be performed using appropriate computer programs (Olszewski, Proteins 25 (1996), 286-299; Hoffman, Comput. Appl. Biosci. 11 (1995), 675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of the polypeptide of the invention and its possible receptor, its ligand or other interacting proteins by computer assistant searches for complementary peptide sequences (Fassina, Immunomethods 5 (1994), 114-120. Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptide mimetics of the protein of the invention or fragments thereof. Such pseudopeptide analogues of the natural amino acid sequence of the protein may very efficiently mimic the parent protein (Benkirane, J. Biol. Chem. 271 (1996), 33218-33224). For example, incorporation of easily available achiral ω-amino acid residues into a protein of the invention or a fragment thereof results in the substitution of amide bonds by polymethylene units of an aliphatic chain, thereby providing a convenient strategy for constructing a peptide mimetic (Banerjee, Biopolymers 39 (1996), 769-777). Superactive peptidomimetic analogues of small peptide hormones in other systems are described in the prior art (Zhang, Biochem. Biophys. Res. Commun. 224 (1996), 327-331). Appropriate peptide mimetics of the protein of the present invention can also be identified by the synthesis of peptide mimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, a three-dimensional and/or crystallographic structure of the polypeptide of the invention can be used for the design of peptide mimetic inhibitors of the biological activity of the polypeptide of the invention (Rose, Biochemistry 35 (1996), 12933-12944; Rutenber, Bioorg. Med. Chem. 4 (1996), 1545-1558).

The structure-based design and synthesis of low-molecular-weight synthetic molecules that mimic the activity of the native biological polypeptide is further described in, e.g., Dowd, Nature Biotechnol. 16 (1998), 190-195; Kieber-Emmons, Current Opinion Biotechnol. 8 (1997), 435-441; Moore, Proc. West Pharmacol. Soc. 40 (1997), 115-119; Mathews, Proc. West Pharmacol. Soc. 40 (1997), 121-125; Mukhija, European J. Biochem. 254 (1998), 433-438.

It is also well known to the person skilled in the art, that it is possible to design, synthesize and evaluate mimetics of small organic compounds that, for example, can act as a substrate or ligand to a cancer-associated HERV-H polypeptide of the invention or the related polypeptide. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.

The nucleic acid molecule of the invention can also serve as a target for activators and inhibitors. Activators may comprise, for example, proteins that bind to the mRNA of a gene encoding a polypeptide of the invention, thereby stabilizing the native conformation of the mRNA and facilitating transcription and/or translation, e.g., in like manner as Tat protein acts on HIV-RNA. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical interest, and for identifying unknown RNA targets for use in treating a disease. These methods and compositions can be used in screening for identifying compounds useful to alter expression levels of proteins encoded by a nucleic acid molecule. Alternatively, for example, the conformational structure of the RNA fragment which mimics the binding site can be employed in rational drug design to modify known antibiotics to make them bind more avidly to the target. One such methodology is nuclear magnetic resonance (NMR), which is useful to identify drug and RNA conformational structures. Still other methods are, for example, the drug design methods as described in WO 95/35367, US-A-5,322,933, where the crystal structure of the RNA fragment can be deduced and computer programs are utilized to design novel binding compounds.

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Furthermore, genes encoding a putative regulator of a cancer-associated HERV-H polypeptide of the invention and/or which excert their effects up- or downstream of a polypeptide of the invention may be identified using, for example, insertion mutagenesis using, for example, gene targeting vectors known in the art. Said compounds can also be functional derivatives or analogues of known inhibitors or activators. Such useful compounds can be for example transacting factors which bind to a cancer-associated HERV-H polypeptide of the invention polypeptide or regulatory sequences of the gene encoding it. Identification of transacting factors can be carried out using standard methods in the art (see, e.g., Sambrook, supra, and Ausubel, supra). To determine whether a protein binds to the protein itself or regulatory sequences, standard native gel-shift analyses can be carried out. In order to identify a transacting factor which binds to the protein or regulatory sequence, the protein or regulatory sequence can be used as an affinity reagent in standard protein purification methods, or as a probe for screening an expression library. The identification of nucleic acid molecules which encode polypeptides which interact with the polypeptids of the invention described above can also be achieved, for example, as described in Scofield (Science 274 (1996), 2063-2065) by use of the so-called yeast "two-hybrid system". In this system the polypeptide encoded by a nucleic acid molecule according to the invention or a smaller part thereof is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express proteins or peptides thereof fused to an activation domain. Thus, if a peptide encoded by one of the cDNAs is able to interact with the fusion peptide comprising a peptide of a polypeptide of the invention, the complex is able to direct expression of the reporter gene. In this way the nucleic acid molecules according to the invention and the encoded peptide can be used to identify peptides and proteins interacting with a HERV-H protein of the invention. It is apparent to the person skilled in the art that this and similar systems may then further be exploited for the identification of inhibitors of the binding of the cancer-associated HERV-H polypeptides of the invention.

Once the transacting factor is identified, modulation of its binding to or regulation of expression of a polypeptide of the invention can be pursued, beginning with, for example, screening for inhibitors against the binding of the transacting factor to the protein of the present invention. Activation or repression of said proteins could then be achieved in animals by applying the transacting factor (or its inhibitor) or the gene encoding it, e.g. in an expression vector. In addition, if the active form of the transacting factor is a dimer, dominant-negative mutants of the transacting factor could be made in order to inhibit its activity. Furthermore, upon identification of the transacting factor, further components in the pathway leading to activation (e.g. signal transduction) or repression of a gene involved in the control of a cancer-associated HERV-H polypeptide of the invention then can be identified. Modulation of the activities of these components can then be pursued, in order to develop additional drugs and methods for modulating the metabolism of protein degradation in animals. Thus, the present invention also relates to the use of the two-hybrid system as defined above for the identification of activators or inhibitors of a cancer-associated HERV-H polypeptide of the invention.

The compounds isolated by the above methods also serve as lead compounds for the development of analog compounds. The analogs should have a stabilized electronic configuration and molecular conformation that allows key functional groups to be presented to the polypetide of the present invention or its possible receptor in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above.

Once the described compound has been identified and obtained, it is preferably provided in a therapeutically acceptable form.

Accordingly, the present invention also relates to a pharmaceutical composition comprising a nucleic acid molecule, polypeptide, recombinant vector, an antisense RNA sequence, a ribozyme, antibody, activator/agonist, inhibitor/antagonist and/or binding partner according to the present invention and a pharmaceutically acceptable excipient, diluent or carrier. In a preferred embodiment, the pharmaceutical composition is a vaccine.

Moreover, the present invention relates to the use of one or more of the compounds of the present invention described above for the preparation of a pharmaceutical composition for the treatment of cancer, preferably colon cancer.

Polypeptides of the present invention that comprise an immunogenic portion of a tumor protein may generally be used for (immuno)therapy of cancer, wherein the polypeptide stimulates the patient's own immune response to tumor cells. A patient may be afflicted with disease, or may be free of detectable disease. Accordingly, the compounds disclosed herein may be used to treat cancer or to inhibit the development of cancer. The compounds are preferably administered either prior to or following surgical removal of primary tumors and/or treatment by administration of radiotherapy and conventional chemotherapeutic drugs.

Pharmaceutical compositions may comprise one or more polypeptides, each of which may contain one or more of the above sequences (or variants thereof), and a physiologically acceptable carrier. The vaccines may comprise one or more such polypeptides and a non-specific immune-response enhancer, wherein the non-specific immune response enhancer is capable of eliciting or enhancing an immune response to an exogenous antigen. Examples of non-specific-immune response enhancers include adjuvants, biodegradable microspheres (e.g., polylactic galactide) and liposomes (into which the polypeptide is incorporated). Pharmaceutical compositions and vaccines may also contain other epitopes of tumor antigens, either incorporated into a fusion protein as described above (i.e., a single polypeptide that contains multiple epitopes) or present within a separate polypeptide.

Alternatively, a pharmaceutical composition or vaccine may contain nucleic acids encoding one or more of the above polypeptides and/or fusion proteins, such that the polypeptide is generated in situ. In such pharmaceutical compositions and vaccines, the nucleic acids may be present within any of a variety of delivery systems known to those of ordinary skill in the art, including nucleic acid expression systems, bacteria and viral expression systems. Appropriate nucleic acid expression systems contain the necessary nucleic acid sequences for expression in the patient (such as a suitable promoter). Bacterial delivery systems involve the administration of a bacterium (such as Bacillus-Calmette-Guerrin) that expresses an epitope of a cell antigen on its cell surface. In a preferred embodiment, the nucleic acid may be introduced using a viral expression system (e.g., vaccinia or other pox virus, retrovirus, or adenovirus), which may involve the use of a non-pathogenic (defective), replication competent virus. Suitable systems are disclosed, for example, in Fisher-Hoch et al., PNAS 86:317-321, 1989; Flexner et al., Ann. N.Y. Acad Sci. 569:86-103, 1989; Flexner et al., Vaccine 8:17-21, 1990; U.S. Pat. Nos. 4,603,112, 4,769,330, and 5,017,487; WO 89/01973; U.S. Pat. No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993. Techniques for incorporating nucleic acids into such expression systems are well known to those of ordinary skill in the art. The nucleic acid may also be "naked," as described, for example, in published PCT application WO 90/11092, and Ulmer et al., Science 259:1745-1749, 1993, reviewed by Cohen, Science 259:1691-1692, 1993. The uptake of naked nucleic acid may be increased by coating the nucleic acid onto biodegradable beads, which are efficiently transported into the cells.

Routes and frequency of administration, as well as dosage, will vary from individual to individual and may parallel those currently being used in immunotherapy of other diseases. In general, the pharmaceutical compositions and vaccines may be administered by injection (e.g., intracutaneous, intramuscular, intravenous or subcutaneous), intranasally (e.g., by aspiration) or orally. A suitable dose is an amount of polypeptide or nucleic acid that is effective to raise an immune response (cellular and/or humoral) against tumor cells in a treated patient. A suitable immune response is at least 10-50% above the basal (i.e., untreated) level.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g., polylactic glycolide) may also be employed as carriers for the pharmaceutical compositions of this invention. Suitable biodegradable microspheres are disclosed, for example, in U.S. Pat. Nos. 4,897,268 and 5,075,109.

Any of a variety of immune-response enhancers may be employed in the vaccines of this invention. For example, an adjuvant may be included. Most adjuvants contain a substance designed to protect the antigen from rapid catabolism, such as aluminum hydroxide or mineral oil, and a nonspecific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

Polypeptides disclosed herein may also be employed in adoptive immunotherapy for the treatment of cancer, preferably colon cancer. Adoptive immunotherapy may be broadly classified into either active or passive immunotherapy. In active immunotherapy, treatment relies on the in vivo stimulation of the endogenous host immune system to react against tumors with the administration of immune response-modifying agents (for example, tumor vaccines, bacterial adjuvants, and/or cytokines).

In passive immunotherapy, treatment involves the delivery of biologic reagents with established tumor-immune reactivity (such as effector cells or antibodies) that can directly or indirectly mediate antitumor effects and does not necessarily depend on an intact host immune system. Examples of effector cells include T lymphocytes (for example, CD8+ cytotoxic T-lymphocyte, CD4+ T-helper, tumor-infiltrating lymphocytes), killer cells (such as Natural Killer cells, lymphokine-activated killer cells), B cells, or antigen presenting cells (such as dendritic cells and macrophages) expressing the disclosed antigens. The polypeptides disclosed herein may also be used to generate antibodies or anti-idiotypic antibodies (as in U.S. Pat. No. 4,918,164), for passive immunotherapy.

The predominant method of procuring adequate numbers of T-cells for adoptive immunotherapy is to grow immune T-cells in vitro. Culture conditions for expanding single antigen-specific T-cells to several billion in number with retention of antigen recognition in vivo are well known in the art. These in vitro culture conditions typically utilize intermittent stimulation with antigen, often in the presence of cytokines, such as IL-2, and non-dividing feeder cells. As noted above, the immunoreactive polypeptides described herein may be used to rapidly expand antigen-specific T cell cultures in order to generate sufficient number of cells for immunotherapy. In particular, antigen-presenting cells, such as dendritic, macrophage or B-cells, may be pulsed with immunoreactive polypeptides or transfected with a nucleic acid sequence(s), using standard techniques well known in the art. For example, antigen presenting cells may be transfected with a nucleic acid sequence, wherein said sequence contains a promoter region appropriate for increasing expression, and can be expressed as part of a recombinant virus or other expression system. For cultured T-cells to be effective in therapy, the cultured T-cells must be able to grow and distribute widely and to survive long term in vivo. Studies have demonstrated that cultured T-cells can be induced to grow in vivo and to survive long term in substantial numbers by repeated stimulation with antigen supplemented with IL-2 (see, for example, Cheever, M., et al, "Therapy With Cultured T Cells: Principles Revisited," Immunological Reviews, 157:177, 1997).

The polypeptides disclosed herein may also be employed to generate and/or isolate tumor-reactive T-cells, which can then be administered to the patient. In one technique, antigen-specific T-cell lines may be generated by in vivo immunization with short peptides corresponding to immunogenic portions of the disclosed polypeptides. The resulting antigen specific CD8+ CTL clones may be isolated from the patient, expanded using standard tissue culture techniques, and returned to the patient.

Alternatively, peptides corresponding to immunogenic portions of the polypeptides of the invention may be employed to generate tumor reactive T-cell subsets by selective in vitro stimulation and expansion of autologous T-cells to provide antigen-specific T-cells which may be subsequently transferred to the patient as described, for example, by Chang et al. (Crit. Rev. Oncol. Hematol., 22(3), 213, 1996). Cells of the immune system, such as T-cells, may be isolated from the peripheral blood of a patient, using a commercially available cell separation system, such as CellPro Incorporated's (Bothell, Wash.) CEPRATE.TM. system (see U.S. Pat. No. 5,240,856; U.S. Pat. No. 5,215,926; WO 89/06280; WO 91/16116 and WO 92/07243). The separated cells are stimulated with one or more of the immunoreactive polypeptides contained within a delivery vehicle, such as a microsphere, to provide antigen-specific T-cells. The population of tumor antigen-specific T-cells is then expanded using standard techniques and the cells are administered back to the patient.

In another embodiment, T-cell and/or antibody receptors specific for the polypeptides can be cloned, expanded, and transferred into other vectors or effector cells for use in adoptive immunotherapy.

In a further embodiment, syngeneic or autologous dendritic cells may be pulsed with peptides corresponding to at least an immunogenic portion of a polypeptide disclosed herein. The resulting antigen-specific dendritic cells may either be transferred into a patient, or employed to stimulate T-cells to provide antigen-specific T-cells which may, in turn, be administered to a patient. The use of peptide-pulsed dendritic cells to generate antigen-specific T-cells and the subsequent use of such antigen-specific T-cells to eradicate tumors in a murine model has been demonstrated by Cheever et al, Immunological Reviews, 157:177, 1997).

Additionally, vectors expressing the disclosed nucleic acids may be introduced into stem cells taken from the patient and clonally propagated in vitro for autologous transplant back into the same patient.

Monoclonal antibodies of the present invention may also be used as therapeutic compounds in order to diminish or eliminate tumors. The antibodies may be used on their own (for instance, to inhibit metastases) or coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

The present invention also relates to a method for detecting cells expressing a HERV-H polypeptide of the present invention. Such cells may comprise neoplastic cells, tumor cells, precursor cells of tumors or, cells showing a disposition to tumors. Tumors, as used in the context of the present invention, may comprise neoplasms such as benign and malignant tumors, carcinomas or dysplasias. In a preferred embodiment of the present invention the tumor is a tumor of the gastrointestinal tract, anogenital tumors, tumor of the urinary tract, tumor of the head and the neck, tumor of the skin, tumor of the respiratory tract, etc.. Tumors may comprise neoplasms such as benign and malignant tumors, carcinomas or dysplasias. In a preferred embodiment the tumor is a carcinoma of the anogenital tract, a carcinoma of the respiratory tract, a carcinoma of the gastrointestinal tract or a carcinoma of the skin. In the most preferred embodiment of the invention the carcinoma is cervical cancer, colon cancer, etc.. The method may be employed for diagnosis, prognosis and/or for monitoring the progression of tumors.

Diagnosis as used in the context of the present invention may comprise determining the level of HERV-H in a sample. Based upon the determined level of HERV-H in the samples individuals can be subdivided into subgroups. The subgroups may be created according to clinical data, such as e.g. survival, recurrence of disease, frequency of metastases etc., related to the particular level of HERV-H determined in the samples. Based upon these subgroups for example an assessment of prognosis may be done. According to the subgroups the therapy of the individuals affected by the tumors may be tailored.Monitoring may comprise detecting the level of HERV-H in samples taken at different points in time and determining the changes in said level. According to said changes the course of the disease can be followed. The course of the disease may be used to select therapy strategies for the particular individual.

Another aspect of diagnosis and monitoring of the disease course according to the present invention may comprise the detection of minimal residual disease. This may comprise for example the detection of a HERV-H level in one or more body samples following initial therapy of an individual once or at several timepoints. According to the level of HERV-H detected in the samples one may select a suitable therapy for the particular individual.

The method for detecting cells expressing HERV-H molecules according to the present invention comprise the following steps:
(a) contacting a biological sample obtained from a patient with a probe that is capable of specifically binding to a nucleic acid molecule or a polypeptide according to the present invention and
(b) determining in the sample an amount of nucleic acid molecules or polypeptide that binds to said probe.

Preferably, steps (a) and (b) are repeated.

As used herein, suitable "biological samples" may comprise any sample comprising cells or cell debris. Biological samples may comprise samples of clinical relevance, such as e.g. secretions, smears, body fluids, urine, semen, stool, bile, biopsies, cell- and tissue-samples. Biopsies as used in the context of the present invention may comprise e.g. resection samples of tumors, tissue samples prepared by endoscopic means or needle biopsies of organs. Furthermore any sample potentially containing the marker molecules to be detected may be a sample according to the present invention. Such samples may comprise for example intact cells, lysed cells or any liquids containing proteins, peptides or nucleic acids. Even solids, to which cells, cell fragments or marker molecules, such as HERV-H nucleic acids or HERV-H proteins, may adhere may be samples according to the present invention. Such solids may comprise for example membranes, glass slides, beads etc..

Preparation of a sample may comprise e.g. obtaining a sample of a tissue, a body fluid, of cells, of cell debris from a patient. According to the present invention preparation of the sample may also comprise several steps of further preparations of the sample, such as preparation of dissections, preparation of lysed cells, preparation of tissue arrays, isolation of polypeptides or nucleic acids, preparation of solid phase fixed peptides or nucleic acids or preparation of beads, membranes or slides to which the molecules to be determined are coupled covalently or non-covalently.

When the target is a nucleic acid molecule, e.g. mRNA, the reagent is typically a nucleic acid probe or a primer for PCR. The person skilled in the art is in a position to design suitable nucleic acid probes based on the nucleic acid sequence provided in Figure 1.

When the target is a polypeptide, the reagent is typically a binding agent like an antibody probe. Polypeptides and fusion proteins of the present invention may be used to generate binding agents, such as antibodies or fragments thereof, that are capable of detecting human tumors, preferably human gastrointestinal and anogenital tumors. Binding agents of the present invention may generally be prepared using methods known to those of ordinary skill in the art, including the representative procedures described herein. Binding agents are capable of differentiating between patients with and without cancer, using the representative assays described therein. The representative assays described below, such as the two-antibody sandwich assay, may generally be employed for evaluating the ability of a binding agent to detect tumors.

The ability of a polypeptide prepared as described herein to generate antibodies capable of detecting primary or metastatic human tumors may generally be evaluated by raising one or more antibodies against the polypeptide (using, for example, a representative method described herein) and determining the ability of such antibodies to detect such tumors in patients. This determination may be made by assaying biological samples from patients with and without primary or metastatic cancer for the presence of a polypeptide that binds to the generated antibodies. Such test assays may be performed, for example, using a representative procedure described below. Polypeptide specific antibodies may be used alone or in combination to improve sensitivity.

There are a variety of assay formats known to those of ordinary skill in the art for using a probe to detect polypeptide markers in a sample; see, e.g., Harlow and Lane, Antibodies: A Laboratory Manual, Cold Spring Harbor Laboratory, 1988. In a preferred embodiment, the assay involves the use of a probe (binding partner) immobilized on a solid support to bind to and remove the polypeptide from the remainder of the sample. The bound polypeptide may then be detected using a second probe (binding partner) that contains a reporter group. Suitable second binding partners include antibodies that bind to the binding partner/polypeptide complex. Alternatively, a competitive assay may be utilized, in which a polypeptide is labeled with a reporter group and allowed to bind to the immobilized binding partner after incubation of the binding partner with the sample. The extent to which components of the sample inhibit the binding of the labeled polypeptide to the binding partner is indicative of the reactivity of the sample with the immobilized binding partner.

The solid support may be any material known to those of ordinary skill in the art to which the antigen may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Pat. No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (see, e.g., Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a second antibody (containing a reporter group) capable of binding to a different site on the polypeptide is added. The amount of second antibody that remains bound to the solid support is then determined using a method appropriate for the specific reporter group. The second antibody, which contains a reporter group, may then be added to the solid support. Preferred reporter groups include enzymes (such as horseradish peroxidase), substrates, cofactors, inhibitors, dyes, radionuclides, luminescent groups, fluorescent groups and biotin. The conjugation of antibody to reporter group may be achieved using standard methods known to those of ordinary skill in the art.

In a related embodiment, the assay is performed in a flow-through or strip test format, wherein the antibody is immobilized on a membrane, such as nitrocellulose. In the flow-through test, polypeptides within the sample bind to the immobilized antibody as the sample passes through the membrane. A second, labeled antibody then binds to the antibody-polypeptide complex as a solution containing the second antibody flows through the membrane. The detection of bound second antibody may then be performed as described above. In the strip test format, one end of the membrane to which antibody is bound is immersed in a solution containing the sample. The sample migrates along the membrane through a region containing second antibody and to the area of immobilized antibody. Concentration of second antibody at the area of immobilized antibody indicates the presence of cancer. Typically, the concentration of second antibody at that site generates a pattern, such as a line, that can be read visually. The absence of such a pattern indicates a negative result.

In another embodiment, the above polypeptides may be used as markers for the progression of cancer, preferably colon cancer. In this embodiment, assays as described above for the diagnosis of colon cancer may be performed over time, and the change in the level of reactive polypeptide(s) evaluated. In general, cancer is progressing in those patients in whom the level of polypeptide detected by the binding agent increases over time. In contrast, colon cancer is not progressing when the level of reactive polypeptide either remains constant or decreases with time. Antibodies for use in the above methods may be prepared by the methods described above.

In another preferred embodiment the diagnostic method is carried out by determining the presence and/or the level of a nucleic acid molecule of the invention indicative for e.g. tumors by using as probes nucleic acid molecules which specifically hybridize to nucleic acid molecules of the invention but not to HERV-sequences present in normal tissues. These nucleic acid molecules can be used, for example, as probes in the diagnostic assay and/or kit described below and, preferably, are oligonucleotides having a length of at least 10, in particular of at least 15 and particularly preferred of at least 50 nucleotides. The nucleic acid molecules and oligonucleotides of the invention can also be used, for example, as primers for a PCR reaction. For example, at least two oligonucleotide primers may be employed in a polymerase chain reaction (PCR) based assay to amplify tumor-specific cDNA derived from a biological sample, wherein at least one of the oligonucleotide primers is specific for a nucleic acid molecule encoding a tumor protein of the present invention. The presence of the amplified cDNA is then detected using techniques well known in the art, such as gel electrophoresis. Similarly, oligonucleotide probes specific for a nucleic acid molecule encoding a tumor protein of the present invention may be used in a hybridization assay to detect the presence of an inventive polypeptide in a biological sample.

One example for the diagnosis of disorders associated with the expression of the HERV-H sequences of the present invention may comprise the detection of auto-antibodies directed against polypeptides encoded by the HERV-H sequences of the present invention. The polypeptides of the present invention may be used to detect the presence of such antibodies in body fluids by methods known to those of skill in the art.

In another preferred embodiment the diagnostic method is carried out to detect disseminated tumor cells in biological samples for example as diagnosis of minimal residual disease. For this purpose the detection of the HERV-H molecules in the body samples may employ generally the same methods as described above for use in diagnosis. For example nucleic acid and/or polypeptide binding agents may be employed to detect the level of the HERV-H sequences of the present invention in body fluids.

For use in research or diagnostic purposes discussed above, kits are also provided by the present invention. Such kits are, eg., useful for the detection of a cancer, preferably colon cancer, said kit comprising at least one probe as discussed above. The probe can be detectably labeled. Such probe may be a specific antibody or specific oligonucleotide. In a preferred embodiment, said kit contains an antibody and allows said diagnosis, e.g., by ELISA and contains the antibody bound to a solid support, for example, a polystyrene microtiter dish or nitrocellulose paper, using techniques known in the art. Alternatively, said kit is based on a RIA and contains said antibody marked with a radioactive isotope. In a preferred embodiment of the kit of the invention the antibody is labeled with enzymes, fluorescent compounds, luminescent compounds, ferromagnetic probes or radioactive compounds.

The following Examples illustrate the invention.

### EXAMPLE 1

### Detection and identification of a HERV-H peptide of the present invention using an expression library constructed from samples of adenocarcinomas of the colon

The copy of the HERV-H sequence disclosed herein may be identified as follows:

Samples of colon adenocarcinoma are used to construct cDNA expression libraries. For this purpose mRNA is isolated form the samples using an mRNA isolation kit (Stratagene, LaJolla California). cDNA libraries are constructed using 5-8 µg of poly (A) RNA using oligo(dT) primers for the first strand synthesis Following the cDNA synthesis the cDNA is cloned directionally into the bacteriophage expression vector lambdaZAPII (Stratagene, LaJolla, California), packaged into particles, and used to tranfect E. coli. Each library results in at lieast 1,5 x 106 primary recombinants

E. coli transfected with recombinant, lambdaZAPII phages (Stratagene, LaJolla, California) are plated onto Luria-Bertani agar plates. Expression of recombinant proteins is induced with isopropyl beta-D-thiogalactoside. Plates are incubated at 37°C until plaques are visible and then blotted onto nitrocellulose membranes. The membranes are blocked with 5% (wt/vol) low-fat milk in Tris-buffered saline and incubated with a 1:1000 dilution of the mouse-serum, which has been preabsorbed with transfected E. coli.

Immunoscreening for the detection of reactive clones in each library is performed with serum from mice, which are immunized with protein extracts from colon carcinomas. Serum antibodies binding to recombinant proteins expressed in lytic plaques are detected by incubation with an alkaline phosphatase-conjugated goat anti-mouse IgG (DAKO, Glostrup, Danmark) and visualized by staining with 5-bromo-4-chloro-3-indolyl phosphate and nitroblue tetrazolium.

Positive clones are subcloned to monoclonality and submitted to in vivo excision of pBluescript phagemids (Stratagene, LaJolla, California). The nucleotide sequence of cDNA inserts is determined using a T7 sequencing kit.

The novel member of the HERV (human endogenous retrovirus)-H family located on Chr. Xp identifiable according to the above protocol shows the following properties:

The major part of env is deleted as compared to published full length HERV-H sequences (Hirose,Y., Takamatsu,M. and Harada,F, Virology 192 (1), 52-61 (1993)). The same pattern of deletion is found in a HERV-H copy derived from chromosome 6 (PAC 271G9 (PAC library described in Ioannou A.P. et al, Nature Genet 6, 84-89, (1994))). The viral gag, pol and env genes are defective in the isolated HERV-H sequence but, in contrast to the other sequences, it containes a new, up to now not described open reading frame (ORF) in the 5' region. This 5' ORF starts directly after the LTR and uses the second ATG after the transcription initiation site (see Tables 1 and 2).

### EXAMPLE 2

### Examination of the expression of the HERV-H sequences of the present invention by RT PCR

The expression of the novel HERV-H copy within a sample may be examined using RT-PCR amplification of the particular mRNA.

In order to specifically amplify transcripts derived from the Xp locus harbouring the HERV-H open reading frame, a novel RT-PCR system has to be developed as follows. One µg of total RNA is treated with DNAse to avoid genomic contamination After inactivation of DNAse, reverse transcription is performed using a specific oligonucleotide primer (5'-ATG GGA CAC GGC TTA GGA G-3'). Five µl of the RT assay are used in PCR analysis. The forward primer (5'-TCA CAG ACT GGG AAG GCA G-3') is located in the open reading frame, the reverse primer (5'-AGG GGT TTG GGG TTT CTT G-3') is located downstream of the ORF-Stop codon. PCR conditions are set as follows: 3 minutes denaturation at 94 °C, followed by 32 cycles of 30 seconds denaturation at 94°C, 30 seconds of annealing at 58°C and 60 seconds of elongation at 72°C. Finally, a 5 minute elongation step at 72°C is added. Amplification products are loaded on ethidium bromide stained agarose gels.

When available, matched pairs of tumor and corresponding normal tissue are analyzed. In total, 5/14 colon cancers analyzed by standard RT-PCR show a differential activation of the HERV-H ORF sequence with a stronger signal in the tumor sample.
The specific sequence from the novel HERV-H disclosed herein is transcribed and encodes a novel protein. To determine whether the sequence of the encoded protein shows any significant homology to a known proteins, online searches were performed using the existing databases, known to those of skill in the art.

### Analysis of the predicted protein:

A database comparison of the peptide sequence using protein BLAST at the NCBI homepage
(http://www.ncbi.nlm.nih.gov/blast/Blast.cgi) did not show any similarity to known proteins.
A Prosite Scan (http://www.expasy.ch/cgi-bin/scanprosite/scanprosite?1) of the translated open reading frame revealed a leucine zipper sequence from position 216 to 237 (LYPFSAFLGQGQVPLNPFSFTL).
A Peptide Mass Scan (http://www.expasy.ch/cgi-bin/peptide-mass.pl) of the translated open reading frame predicted a peptide mass of 29658.22 Dalton and a theoretical pI of 10.35. 
Primers used for standard RT-PCR
(58°C, 35 cycles):

RT
ATG GGA CAC GGC TTA GGA G (retrobreakrev2)

PCR
TCA CAG ACT GGG AAG GCA G (retrocdrfor)
AGG GGT TTG GGG TTT CTT G (retro1421rev)
 
Taqman-Primer
463 CACGTTTTATCCGTGGACCC
464 AGGCATCCCTGCAATGATTAA
465 CCGGCGCTGGTCACAG
466 TGAAACGTGGGTGTATAATCAGAGA
 Used: 463 + 464 or 465 + 466; both combinations showed the same results.

### Example 3

### Stimulation of CD8+ T cells against the HERV-H peptide of the present invention.

The concept of this study is to stimulate cultured lymphocytes with specific antigens. Peripheral mononuclear blood cells (PMBC) from a healthy donor are obtained and an Elispot analysis is performed. Cytokines are released by activated lymphocytes that bind to specific antibodies coated on the culture vessel. After washing the culture vessels, cytokines can be detected using a second antibody.

PBLs from a HLA-A0201 positive healthy donor are purified using a Ficoll Paque gradient. T-lymphocytes are separated from B-lymphocytes and monocytes using antibody coupled magnetobeads (CD11, CD16, CD19, CD36 and CD56) (Pant T cell isolation kit, Milteny, Bergisch Gladbach, Germany) . 2x107 T-cells are extracted from 30 ml blood.

The following HLA-A0201 restricted peptides from the HERV-H ORF are identified using the NIH peptide predicition software (NIH bioinformation software http://bimas.dcrt.nih.gov.cgi-bin/molbio/ken parker comboform) :

9mer peptides:
LLSVSLPLL
SLRVSTPSL
CLYPFSÄFL
FLLSSPTSL
NLVSSLFLL

1omer peptides:
ILRPPALCSL
ALVTDWEGSL
PLLSVSLPLL
SLNFNSFHFL

Isolated T-cells are incubated with T2-cells, that are (a) either loaded with the 9mer mix or (b) with the 10mer mix (10µg/peptide). The T-cells are restimulated weekly over a course of six weeks. 107 T-cells are cultivated in 24well plates together with 2x106 peptide loaded T2 cells.
The reactivity of T-cells against peptide loaded T2-cells is determined by IFN-γ Elispot analysis once in every week, starting on day 0 of the experiment. On day 28, a reactivity of the 9mer as well as the 10mer mix is observed. Very strong reactions are seen with the 9mer CLYPFSAFL-peptide and with the 10mer FLLSSPTSLT peptide. A weaker reaction is observed with the 10mer SLNFNSFHFL peptide.

These experiments show that CD8+ T cells can be stimulated against the novel HERV-H protein.

### Example 4

### Determination of the level of expression of the HERV-H ORF in different cell lines

A series of cell lines are used to determine the expression of HERV-H sequences of the present invention

The determination of the level of expression is performed as given in Example 2 by RT-PCR using the primers given there.

Analysis of the amplificates by agarose gel electrophoresis shows expression of HERV-H sequences of the present invention in four cell lines. The cell lines comprise three cell lines derived from colon cancer and one cell line derived from cervical cancer.

### Example 5

### Multiple alignment of HRV-H sequences of the present invention to other HERV-H sequences

Sequence comparison studies are performed, to identify the similarity between the HERV-H sequences of the present invention and other members of the HERV-H family. The alignment of four different sequences is shown in figure 6. There is a high degree of similarity between the shown sequences. Irrespective of the similarity between the sequences only the sequence disclosed herein shows an ORF.

### Example 6

### Detection of the expression of HERV-H sequences of the present invention in carcinomas and adenomas of the colon using RT-PCR

Samples of coloncarcinomas and colon adenomas are used to determine the level of mRNA coding for HERV-H sequences of the present invention using semi-quantitative RT PCR. Five samples from carcinomas and 37 samples from adenomas are used in this study.

mRNA is isolated from the samples and patient-matched normal colon mucosa using Qiagen reagents (Qiagen, Hilden, Germany), and single-stranded cDNA is synthesized using Superscript II (Life Technologies, Inc.). Quantitative PCR is performed using the 7700 Sequence Detector (TaqmanTM) and the SYBR Green PCR Master-Mix, as described in the manufacturers manual (Applied Biosystems, Foster City, CA).

PCR reactions are performed in 25 µl volumes with a final concentration of 300 nmol for each primer, with 95°C for 15 sec and 60°C for 60 sec, for 40 cycles. The amplification was performed using the same primers as given in Example 2 for the TaqMan analysis. The specificity of the PCR products is verified by gel electrophoresis (data not shown).

It turns out, that HERV-H sequences of the present invention are strongly overexpressed as well in a certain percentage of carcinomas as in a percentage in adenomas. Using a cut-off value of about 4-fold overexpression, the HERV-H sequences of the present invention is at least overexpressed in one third of the samples of adenomas of the colon in comparison to half of the samples of carcinomas.

The results show, that HERV-H sequences of the present invention are expressed as well in carcinomas as in precursors. Thus the HERV-H sequences of the present invention may be useful even in early diagnosis of precursors of carcinomas.

## Claims

1. A nucleic acid molecule encoding a cancer-associated HERV-H polypeptide or a polypeptide exhibiting a biological property of a cancer-associated HERV-H polypeptide and being selected from the group consisting of
(a) a nucleic acid molecule encoding a polypeptide that comprises the amino acid sequence as depicted in Figure 2;
(b) a nucleic acid molecule comprising the coding region of the nucleotide sequence as depicted in Figure 1;
(c) a nucleic acid molecule included in DSMZ Deposit No. DSM 14799;
(d) a nucleic acid molecule the nucleic acid sequence of which deviates from the nucleic sequence specified in (b) to (c) due to the degeneration of the genetic code; and
(e) a nucleic acid molecule, which encodes a polypeptide that comprises a fragment or variant of the amino acid sequence depicted in Figure 2.

2. A recombinant vector containing the nucleic acid molecule of claim 1.

3. The recombinant vector of claim 2 wherein the nucleic acid molecule is operatively linked to regulatory elements allowing transcription and synthesis of a translatable RNA in prokaryotic and/or eukaryotic host cells.

4. A recombinant host cell which contains the recombinant vector of claim 3.

5. The recombinant host cell of claim 4, which is a mammalian cell, a bacterial cell, an insect cell or a yeast cell.

6. A polypeptide exhibiting a biological property of the cancer-associated HERV-H polypeptide which is encoded by a nucleic acid molecule of claim 1.

7. A method of producing a polypeptide exhibiting a biological property of the cancer-associated HERV-H polypeptide comprising:
(a)culturing the recombinant host cell of claim 4 or 5 under conditions such that said polypeptide is expressed; and
(b) recovering said polypeptide.

8. The polypeptide produced by the method of claim 7.

9. The polypeptide of claim 6 or 8, which is fused to a tumor antigen.

10. An antisense RNA sequence **characterized in that** it is complementary or reverse-complementary to an mRNA transcribed from a nucleic acid molecule of claim 1 or a part thereof and can selectively bind to said mRNA or part thereof, said sequence being capable of inhibiting the synthesis of the polypeptide encoded by said nucleic acid molecule.

11. A ribozyme **characterized in that** it is complementary or reverse-complementary to an mRNA transcribed from a nucleic acid molecule of claim 1 or a part thereof and can selectively bind to and cleave said mRNA or part thereof, thus inhibiting the synthesis of the protein encoded by said nucleic acid molecule.

12. A binding agent directed against and specifically recognizing the polypeptide of claim 6 or 8.

13. The binding agent of claim 12 which is an antibody.

14. A method for identifying a binding partner to a polypeptide of claim 6 or 8 comprising:
(a) contacting a polypeptide of claim 6 or 8 with a compound to be screened; and
(b) determining whether the compound effects an activity of said polypeptide or whether binding of the compound to said polypeptide has occurred.

15. A method for identifying activators/agonists or inhibitors/antagonists of the polypeptide of claim 6 or 8 comprising the steps of:
(a) incubating a candidate compound with a polypeptide of claim 6 or 8;
(b) assaying a biological activity, and
(c) determining if a biological activity of said polypeptide has been altered.

16. A method for identifying activators or inhibitors of the expression of the polypeptide of claim 6 or 8 comprising the steps of:
(a) incubating a candidate compound with a in-vitro or in-vivo test system or administering a test compound to a test organism;
(b) detecting the level of the polypeptide within the test system, and
(c) determining if the level of said polypeptide has been altered.

17. A method of identifying and obtaining a drug candidate for therapy of a cancer comprising the steps of:
(a) contacting the polypeptide of claim 6 or 8 or a cell expressing said polypeptide in the presence of components capable of providing a detectable signal in response to interaction of said polypeptide with said drug candidate to be screened, and
(b) detecting presence or absence of a signal or increase of the signal generated, wherein the presence or increase of the signal is indicative for a putative drug.

18. An activator/agonist or inhibitor/antagonist of the polypeptide of claim 6 or 8, an activator or inhibitor of the expression of the polypeptide of claim 6 or 8 or a binding partner of the polypeptide of claim 6 or 8 obtainable by the method of claim 14, 15 or 16.

19. A pharmaceutical composition comprising the nucleic acid molecule of claim 1, the recombinant vector of claim 2 or 3, the polypeptide of claim 6, 8 or 9, the antisense RNA sequence of claim 10, the ribozyme of claim 11, the binding agent of claim 12, the antibody of claim 13 or the activator/agonist or inhibitor/antagonist of the claim 18.

20. The pharmaceutical composition of claim 19 which is a vaccine.

21. Use of the nucleic acid molecule of claim 1, the recombinant vector of claim 2 or 3, the polypeptide of claim 6, 8,or 9, the antisense RNA sequence of claim 10, the ribozyme of claim 11, the binding agent of claim 12, the antibody of claim 13 or the activator/agonist or inhibitor/antagonist of claim 18 for the preparation of a pharmaceutical composition for the treatment of cancer.

22. Use according to claim 21, wherein the cancer is gastrointestinal cancer or anogenital cancer.

23. Use according to claim 21 or 22 wherein the treatment is immunotherapy.

24. A method for detecting a level of HERV-H ORF molecules in a biological sample comprising the following steps:
(a) contacting a biological sample obtained from a patient with a probe that is capable of binding to a nucleic acid molecule according to claim 1 or a polypeptide according to claim 6 or 8; and
(b) determining in the sample an amount of nucleic acid molecules or polypeptide that bind to said probe.

25. The method of claim 24, which is used for the detection of cancer, a precursor of a cancer or a disposition to cancer or for monitoring the progression of cancer in a patient.

26. The method of claim 24 or 25, wherein the probe is at least one nucleic acid molecule.

27. The method of any one of claims 24 to 26 comprising a nucleic acid based amplification reaction.

28. The method of anyone of claims 24 to 27, wherein the probe is a first and a second oligonucleotide primer and wherein a polymerase chain reaction is carried out or wherein the probe is a hybridization probe.

29. The method of claim 24, wherein the probe is the binding agent of claim 12.

30. The method of any one of claims 24 to 29, wherein the probe is detectably labeled.

31. The method of claim 30, wherein the label is selected from the group consisting of a radioisotope, a bioluminescent compound, a chemiluminescent compound, a fluorescent compound, a metal chelate, or an enzyme.

32. The method of any one of claims 24 to 30, which is used in detection of a minimal residual disease.

33. A research kit or diagnostic kit useful for the detection of a level of the novel HERV-H ORF molecules in a sample, said kit comprising at least one oligonucleotide probe and/or an antibody capable of specifically binding to the nucleic acid molecule of claim 1 or the polypeptide of claim 6 or 8.

34. The method of any one of claims 24 to 32 or the kit of claim 33, wherein the cancer is gastrointestinal cancer or anogenital cancer.
